# EUROPÄISCHE PATENTSCHRIFT

(11) **EP 1 891 023 B1**
(45) Veröffentlichungstag und Bekanntmachung des Hinweises auf die Patenterteilung: **14.09.2011**
(21) Anmeldenummer: 06707284.3
(22) Anmeldetag: 27.02.2006
(51) Int. Cl.: C07D 251/60

(54) **VERFAHREN ZUR HERSTELLUNG VON MELAMIN**
METHOD FOR PRODUCING MELAMINE
PROCEDE POUR PRODUIRE DE LA MELAMINE

(30) Priorität: 06.05.2005 DE 102005021084; 13.05.2005 DE 102005023042
(43) Veröffentlichungstag der Anmeldung: 27.02.2008
(73) Patentinhaber: Lurgi AG, 60295 Frankfurt am Main (DE)
(72) Erfinder: MÜLLER-HASKY, Martin, 63150 Heusenstamm (DE); EBERHARDT, Jürgen, 63110 Rodgau (DE); SCHADT, Arne, 61231 Bad Nauheim (DE)
(74) Vertreter: Meyer-Dulheuer, Karl-Hermann
(86) Internationale Anmeldenummer: PCT/EP2006/001769
(87) Internationale Veröffentlichungsnummer: WO 2006/119814

(56) Entgegenhaltungen:
- WO-A-03/080548
- WO-A-2004/016599
- DE-B- 1 204 679
- DE-B- 1 222 506
- GB-A- 1 216 100
- US-A- 3 290 309
- US-A- 3 386 999
- US-A- 3 547 919
- US-A- 3 555 784
- US-A- 3 578 413
- US-A- 3 697 519
- US-A- 4 348 520
- US-A1- 2001 005 751
- G.M. CREWS, W. RIPPERGER, D.B. KERSEBOHM, J. SEEHOLZER, T. GÜTHNER: "Melamine and Guanamines" ULLMANN'S ENCYCLOPEDIA OF INDUSTRIAL CHEMISTRY, WILEY, VCH VERLAG,WEINHEIM, DE, Bd. 21, 2003, Seiten 205-216, XP002393510

## Beschreibung

Die Erfindung betrifft ein Verfahren zur Herstellung von Melamin durch Zersetzung von Harnstoff mit anschließender Desublimation des Melamins aus der Gasphase in einem Kristallisationsapparat.

Die Herstellung von Melamin ausgehend von Harnstoff ist ein lange bekanntes Verfahren, wobei zwei Arten von Prozessführung zu unterscheiden sind: das nicht katalytische Hochdruckverfahren und das katalytische Niederdruckverfahren. Das Hochdruckverfahren erfordert Drucke von mindestens 8 MPa, das katalytische Niederdruckverfahren wird hingegen in einer Wirbelschicht bei einem Druck von 0,1 bis maximal 1 MPa und Temperaturen von mindestens 380 bis 410°C durchgeführt. Das verwendete Trägergas beim Niederdruckverfahren besteht entweder aus Ammoniak oder aus einer Mischung aus Kohlendioxid und Ammoniak, wobei das erhaltene Melamin nach der Reaktion in gasförmiger Form vorliegt.

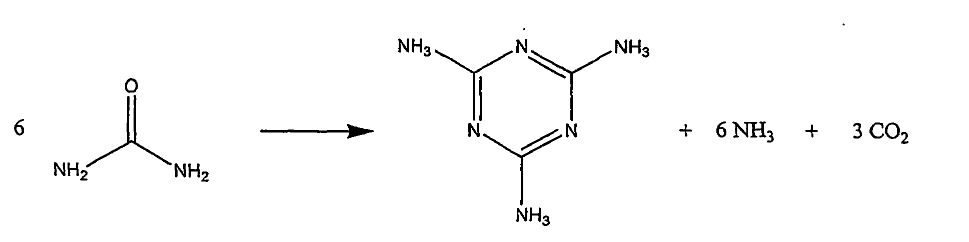

Die Ausbeute an Melamin beträgt so 90 bis 95%, bezogen auf die eingesetzte Menge an Harnstoff. In der Literatur sind die drei häufigsten Niederdruckverfahren bekannt als BASF-Verfahren, Chemie-Linz-Verfahren und Stamicarbon Verfahren.

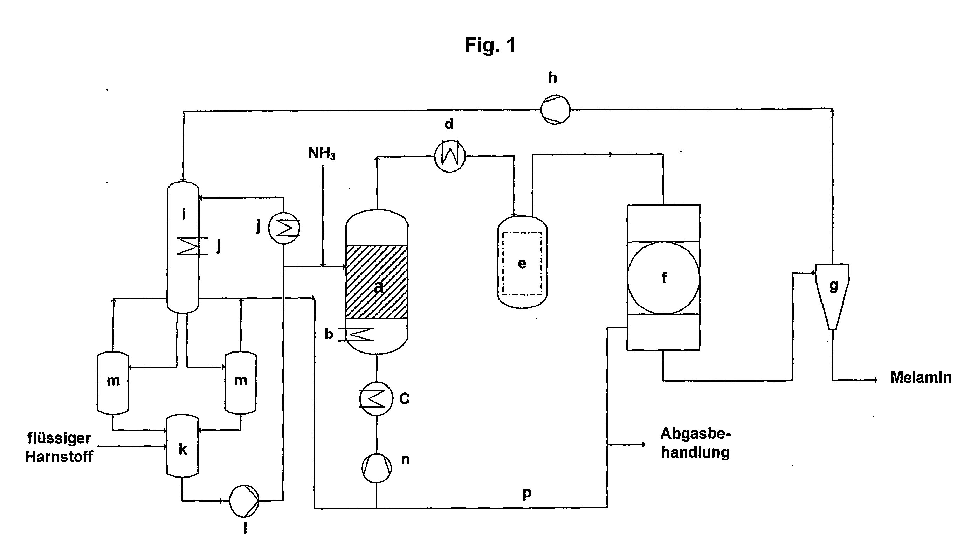

Bei dem BASF-Verfahren handelt es sich um ein einstufiges Reaktionsverfahren (Fig. 1), bei dem geschmolzener Harnstoff bei einer Temperatur von 395 bis 400°C unter nahezu atmosphärischem Druck in einer Wirbelschicht umgesetzt wird. Das hierbei erhaltene Reaktionsgas enthält neben Melamin und Harnstoff zudem Spuren von Nebenprodukten wie Melem und Melam sowie Reaktionsgas bestehend aus Ammoniak und Kohlendioxid. Das entstandene Reaktionsgasgemisch wird anschließend abgekühlt, der ausgetragene Katalysator und die auskristallisierten Nebenprodukte abgetrennt und das melaminhaltige Reaktionsgas in einen Kristallisator eingespeist. Im Kristallisator wird das melaminhaltige und noch heiße Gas mit kalten Reaktionsgas (Quenchgas) abgekühlt, wodurch das melaminhaltige Gas auf Temperaturen von 190 bis 220°C abgekühlt wird. Unter diesen Bedingungen desublimiert Melamin zu annähernd 98% aus dem Reaktionsgas. Nach der Abtrennung des Melamins wird das verbleibende Gas (Kreislaufgas) mit Hilfe eines Kreisgasgebläses zu einer Harnstoffwäsche gefördert, wo es im direkten Kontakt mit der Harnstoffschmelze gekühlt, gewaschen und zurück in den Reaktor geleitet wird. Die Temperatur des Quenchgases liegt bei - 138°C, sodass es zur Einstellung der Temperatur von 190 bis 220°C in der Kristallisationsvorrichtung erforderlich ist, pro Kilogramm melaminhaltiges Gas 2,5 bis 3,5 kg Quenchgas beizumengen.

Die Abtrennung von Melamin aus melaminhaltigen Gasgemischen ist seit langem ein wohlbekannter Prozess. So beschreibt bereits die deutsche Offenlegungsschrift 1 204 679, dass die Abscheidung von Melamin aus einem Gasstrom durch die Kühlung an kalten Wänden, oder durch das Inkontaktbringen mit einem kalten Inertgasstrom, einer kalten inerten Flüssigkeit oder kalten inerten Feststoffen erfolgen kann.

Die Offenlegungsschrift US 4 348 520 A beschreibt die Desublimierung von Melamin aus dem Off-gas an Melaminpartikeln in einem Wirbelbett. Die Kühlung wird dabei indirekt über Wärmeaustauscher realisiert. In der Offenlegungsschrift US 2001/005751 A1 wird ein Hochdruckverfahren zur Melaminsynthese genutzt, wobei flüssiges Melamin entsteht, welches anschließend an festen Melaminpartikeln abgesondert wird und dadurch eine Kühlung erfährt. Die Offenlegungsschrift WO 2004/016599 A beschreibt ein Verfahren; bei dem ein gasförmiges Melamin enthaltender Strom mit einem zweiten Gasstrom in Kontakt gebracht wird, der Melamin in Partikelform enthält. Nach Mischung der Gasströme erfolgt eine Kühlung des Gemisches.

Das gängige Verfahren zur Abtrennung von Melamin durch Inkontaktbringen mit einem kalten Inertgasstrom weist jedoch den Nachteil auf, dass für die erforderliche Temperaturerniedrigung im Verhältnis zum Reaktionsgasvolumen sehr große Quenchgasvolumina erforderlich sind. Das Verhältnis beträgt ca. 4 zu 1. Aufgrund der großen Gasvolumina sind entsprechend groß dimensionierte Apparate erforderlich, was mit entsprechenden finanziellen Mehrkosten bei der Produktion von Melamin und mit einem höheren Investitionsaufwand verbunden ist. Darüber hinaus müssen die den Produktzyklon verlassenen Gase gereinigt und mit Hilfe eines Kreisgasgebläses rezirkuliert werden. Dazu kommt, dass das Kreislaufgas noch melamingesättigt ist und bei jeder weiteren Temperaturerniedrigung Melamin desublimiert. Diese Melaminanhaftungen finden auch an unerwünschten Stellen wie dem Kreisgasgebläse statt, was zu deutlich verkürzten Anlagenbetriebszeiten, zu unerwünschtem Produktionsausfall und erhöhtem Wartungsbedarf führt.

Der Erfindung liegt die Aufgabe zugrunde, ein Verfahren bereit zu stellen, das es gestattet, Melamin in einer kostengünstigen Weise aus melaminhaltigen Reaktionsgasen abzutrennen, ohne den Einsatz von Quenchgas und ohne den Einsatz eines Kompressors n (Fig. 1).

Dieses Problem wird durch das erfindungsgemäße Verfahren zur Herstellung von Melamin gelöst, indem heißes, melaminhaltiges Reaktionsgas aus dem Reaktor zur Desublimation des Melamins in einem Kristallisationsgefäß durch gleichzeitiges Einleiten von kristallinem Melamin abkühlt (gequencht) wird, wobei das zum Quenchen eingesetzte, kristalline Melamin eine Temperatur von weniger als 100°C, vorzugsweise von etwa 40°C, aufweist.

Die mit dieser Erfindung erzielten Vorteile bestehen darin , dass durch die Verwendung von kristallinem Melamin zur Desublimation des gasförmigen Melamins die Menge an Gas, die im Prozess zirkuliert, deutlich reduziert wird, was zu kleineren Apparate- und Rohrleitungsabmessungen führt, was wiederum in geringeren Investitionskosten resultiert.

Darüber hinaus kann der bei Verwendung von Quenchgas erforderliche Kompressor entfallen. Durch Wegfall des Quenchgasleitung p (Fig. 1) kann die reduzierte Kreislaufgasmenge direkt vom bereits vorhandenen Kompressor n (Fig.1) gefördert werden.

Vorteilhafte Ausgestaltungen der Erfindung sind in den Patentansprüchen 2 und folgende dargelegt. Eine bevorzugte Ausführungsform ermöglicht es, dass das melaminhaltige Reaktionsgas im Reaktionsgefäß durch Einleiten von kristallinem Melamin von etwa 350°C auf etwa 190°C abgekühlt wird. Vorzugsweise wird die Temperatur des aus dem Kristallisationsgefäß entnommenen Melamins durch die Menge des zum Quenschen eingesetzten kristallinen Melamins reguliert wird, wobei diese Temperatur nicht unter 190°C liegen soll. Besonders günstig ist es, wenn der überwiegende Teil des aus dem Kristallisationsgefäß entnommenen Melamins im Kreislauf erneut in das Kristallisationsgefäß eingeführt und zum Quenschen der heißen melaminhaltigen Reaktionsgase eingesetzt wird. Es ist weiterhin bevorzugt, Melamin durch flüssiges oder gasförmiges Ammoniak zu desublimieren. In einer vorteilhaften Ausgestaltung werden die melaminhaltigen Reaktionsgase durch das Einleiten von flüssigem Ammoniak von 350°C auf 190°C abgekühlt. Dabei ist es günstig, wenn der verwendete Ammoniak einen Druck von 0,01 bis 0,26 MPa und eine Temperatur von -34 bis +60°C aufweist. Durch die Menge an eingesetztem Ammoniak kann somit die Temperatur des entnommenen Melamins reguliert werden, welche vorzugsweise bei 190°C liegt.

Ausführungsbeispiele der Erfindung sind in den Zeichnungen dargestellt und werden im Folgenden näher beschrieben.

Es zeigen:
- Fig. 1: Verfahren zur Herstellung von Melamin nach dem BASF- Verfahren;
- Fig. 2: erfindungsgemäße Verfahren zur Herstellung von Melamin durch Einleiten von kristallinem Melamin;
- Fig.3: erfindungsgemäße Verfahren zur Herstellung von Melamin durch Einleiten von flüssigem Ammoniak.

Fig. 1 zeigt das Verfahren zur Herstellung von Melamin nach dem BASF-Verfahren. Hierbei wird geschmolzener Harnstoff mittels eines Hamstoffvorratsbehälter k dem System zugeführt. Durch eine Pumpe I wird der Harnstoff der Harnstoffwäsche i zugeführt; in einem nachgeschalteten Tropfenabscheider m werden anschließend die flüssigen und die gasförmigen Bestandteile voneinander getrennt. Ein Teil der hierbei erhaltenen gasförmigen Bestandteile werden durch einen Kompressor n verdichtet, in einem Heizelement c vorgeheizt und anschließend dem Reaktor a zugeführt, wo sie zur Ausbildung der katalytisch wirkenden Wirbelschicht benötigt werden. Die den Reaktor a verlassenden gasförmigen Bestandteile werden durch einen Gaskühler d gekühlt und anschließend einem Gasfilter e zugeleitet. In diesem Gasfilter e werden ausgefallene Nebenprodukte, wie zum Beispiel Melem, Melam und Katalysatoraustrag von dem Reaktionsgas getrennt. Das erhaltene Reaktionsgas wird anschließend einer Kristallisationsvorrichtung f zugeführt, wo dieses mit weiterem Reaktionsgas (Quenchgas), welches vorzugsweise eine Temperatur von ca. 140°C auf weist, auf Temperaturen von 190 bis 200°C gekühlt wird. Das in der Kristallisationsvorrichtung erhaltene kristalline Melamin wird anschließend einem Produktzyklon g zugeführt, im dem der Gasstrom von dem kristallinen Melamin abgetrennt wird. Nach Abtrennung des Melamins wird das Gas mit Hilfe eines Kreisgasgebläses h zur Harnstoffwäsche i befördert.

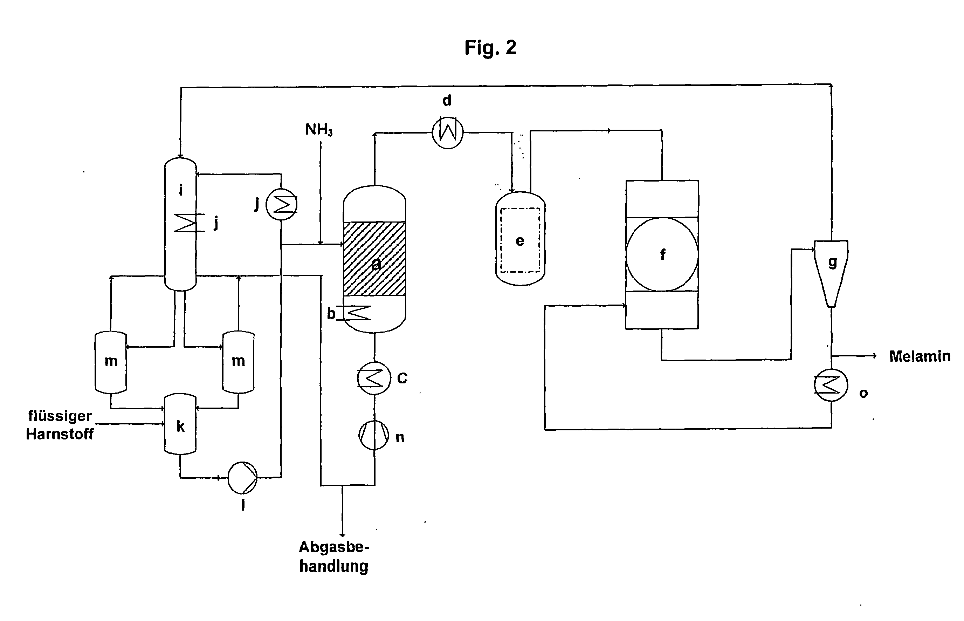

Fig. 2 zeigt ein erfindungsgemäßes Verfahren zur Herstellung von Melamin durch Einleiten von kristallinem Melamin. Flüssiger Harnstoff wird hierbei durch einen Harnstoffvorratsbehälter k dem System zugeführt. Durch eine Pumpe I und Kühleinheit j wird der flüssige Harnstoff der Harnstoffwäsche i zugeführt, wobei anschließend in einem Tropfenabscheider m flüssige und gasförmige Bestandteile voneinander getrennt werden. Die gasförmigen Bestandteile werden anschließend durch einen Kompressor n verdichtet und in einem Gasheizelement c erhitzt; die erhitzten Gase werden anschließend dem Reaktor a zugeführt, wo sie zur Erzeugung der katalytisch wirkenden Wirbelschicht benötigt werden. Die im Reaktor gebildeten gasförmigen Bestandteile werden in einem Gaskühler d gekühlt und anschließend einem Gasfilter e zugeführt, in dem auskristallisierte Bestandteile wie Melem, Melam und Katalysatoraustrag von den gasförmigen Bestandteilen getrennt werden. Die gasförmigen Bestandteile werden anschließend einer Kristallisationsvorrichtung f zugeführt, wobei die gasförmigen Bestandteile durch kristallines Melamin abgekühlt werden. Das hierbei gebildete Melamin wird anschließend durch einen Produktzyklon g von den gasförmigen Bestandteilen abgetrennt. Ein Teil des hierbei gewonnenen Melamins wird in einem Feststoffkühler o auf Temperaturen von ca. 40°C abgekühlt und wieder dem Kristallisationsapparat zugeführt, um das heiße aus dem Reaktor kommende melaminhaltige Gas abzukühlen. Das vom Melamin befreite Gas strömt vom Produktzyklon g ohne weitere Fördereinrichtung zur Harnstoffwäsche i. Die im BASF Verfahren enthaltene Quenchgasleitung p und das Kreislaufgebläse h entfallen.

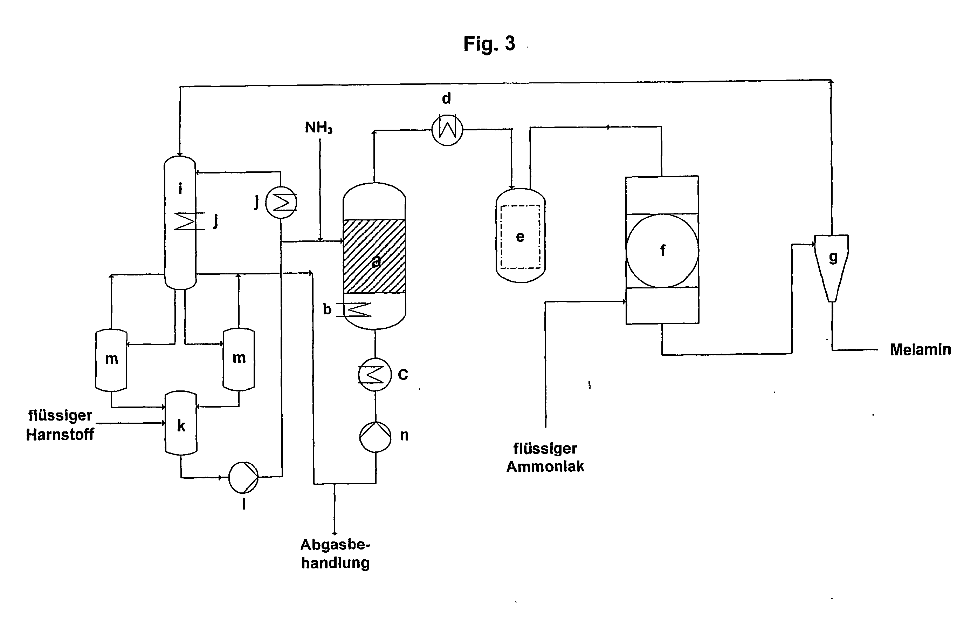

Fig.3 zeigt ein erfindungsgemäßes Verfahren zur Herstellung von Melamin durch Einleiten von flüssigem Ammoniak. Flüssiger Harnstoff wird hierbei durch einen Harnstoffvorratsbehälter k dem System zugeführt. Durch eine Pumpe I und Kühleinheit j wird der flüssige Harnstoff der Harnstoffwäsche i zugeführt, wobei anschließend in einem Tropfenabscheider m flüssige und gasförmige Bestandteile voneinander getrennt werden. Die gasförmigen Bestandteile werden in der für die Reaktion erforderliche Mengen in einen Kompressor n verdichtet, in einem Heizeinheit c erhitzt und anschleißend dem Reaktor a zugeführt. Die im Reaktor gebildeten gasförmigen Bestandteile werden in einem Gaskühler d gekühlt und anschließend einem Gasfilter e zugeführt, in dem auskristallisierte Bestandteile wie Melem, Melam und Katalysatoraustrag von den gasförmigen Bestandteilen getrennt werden. Die gasförmigen Bestandteile werden anschließend einer Kristallisationsvorrichtung f zugeführt, wobei die gasförmigen Bestandteile durch flüssiges, verdampfendes Ammoniak abgekühlt wird. Das hierbei gebildete Melamin wird anschließend durch einen Produktzyk-Ion g von den gasförmigen Bestandteilen abgetrennt. Die vom Melaminfeststoff weitgehend befreiten Gase strömen zurück zur Harnstoffwäsche i.

Die im BASF Verfahren enthaltene Quenchgasleitung p und das Kreislaufgebläse h entfallen auch hier.

Die Erfindung wird anhand der Beispiele 1, 2 und 3 näher beschrieben.

### Beispiel 1

Beispiel 1 beschreibt das bekannte BASF-Verfahren. Der den Reaktor verlassende heiße melaminhaltige Gasstrom hat nach der Zwischenkühlung und Filtration die folgende Zusammensetzung bei einer Temperatur von 320°C.

| | **kmol/h** | **mol%** |
|---|---|---|
| Ammoniak NH₃ | 1709 | 67 |
| Kohlendioxid CO₂ | 740 | 29 |
| Melamin C₃H₆N₆ | 51 | 2 |
| Isocyansäure HNCO | 38 | 1,5 |
| Inert | 31 | 0,5 |
| Summe | 2551 | 100 |

Die erhaltenen melaminhaltigen Gase werden anschließend in der Kristallisationsvorrichtung mit dem Quenchgas in Kontakt gebracht. Das Quenchgas, das typischerweise aus 30 mol-% CO₂ und 70 mol-% NH₃ besteht und eine Temperatur von 138°C aufweist, reduziert die Temperatur am Austritt der Kristallisationsvorrichtung auf bis zu 205°C. Unter Berücksichtigung des Desublimationsenthalpie des Melamins und der spezifischen Wärmekapazität der Gasmischung werden 7650 kmol/h Quenchgas benötigt. Dies entspricht einem Verhältnis von ca. 3 Teilen Quenchgas zu 1 Teil melaminhaltigem Gas. Die Menge des Gases nach der Kristallisationsvorrichtung und nach der Abtrennung des kristallförmigen Melamins beträgt 10200 kmol/h. Die Menge an dampfförmigem Melamin, die bei einer Temperatur von 205°C nicht kristallisiert werden kann, beträgt etwa 0,6 kmol/h.

### Beispiel 2

Beispiel 2 beschreibt das erfindungsgemäße Verfahren zur Desublimation von Melamin durch Quenchen mit Melaminkristallen. Das heiße melaminhaltige Gas mit gleicher Zusammensetzung und gleicher Temperatur wie in Beispiel 1 wird in der Kristallisationsvorrichtung mit gekühltem Melamin zugeführt. Die Temperatur des gekühlten Melamins beträgt 40°C. Unter Berücksichtigung der Desublimationsenthalpie des Melamins, der spezifischen Wärmekapazität der Gasmischung sowie der spezifischen Wärmekapazität des festen Melamins benötigt man für eine Mischtemperatur von 230°C nach der Kristallisationsvor richtung 380 kmol/h Melamin. Die Menge an Gas beträgt nach der Abtrennung des kristallinen Melamins nur noch 2500 kmol/h. Vergleicht man die zirkulierende Gasmenge aus diesem Beispiel mit dem Gasvolumen aus dem Beispiel 2 so unterscheiden sich diese um den Faktor vier. Folglich können die Apparaturen f, g, i und m (Fig. 1 bis 3) und die Verbindenden Rohrleitungen nur noch halb so groß dimensioniert werden. Auf das Kreisgasgebläse h (Fig. 1) kann ganz verzichtet werden.

### Beispiel 3

Beispiel 3 beschreibt das erfindungsgemäße Verfahren zur Desublimation von Melamin mit flüssigem Ammoniak. Der den Reaktor a verlassende melaminhaltige Gasstrom hat nach der Zwischenkühlung und Filtration die nachfolgende typische Zusammensetzung bei einer Temperatur von 320°C.

| | **kmol/h** | **mol%** |
|---|---|---|
| Ammoniak NH₃ | 2066 | 81 |
| Kohlendioxid CO₂ | 383 | 15 |
| Melamin C₃H₆N₆ | 51 | 2 |
| Isocyansäure HNCO | 38 | 1,5 |
| Inert | 13 | 0,5 |
| Summe | 2551 | 100 |

Das heiße melaminhaltige Gas wird in der Kristallisationsvorrichtung mit verdampftem Ammoniak in Kontakt gebracht. Die Temperatur des Ammoniaks beträgt 40°C und der Druck 0,16 MPa. Sollen am Austritt der Kristallisationsvorrichtung die gleichen Mengen an dampfförmigem Melamin wie in Beispiel 1 und 2 vorliegen, so sind 650 kmol/h Ammoniak erforderlich. Die Temperatur, die am Austritt eingestellt wird, beträgt 225°C. Die Menge an Gas, die nach der Abtrennung des kristallinen Melamins zur Harnstoffwäsche strömt, beträgt 3200 kmol/h. Vergleicht man die zirkulierende Gasmenge zwischen diesem Beispiel und Beispiel 1, so stellt sich eine Verkleinerung des Gasvolumens um den Faktor 3 dar. Die Abmessungen folgender Apparate verkleinern sich damit deutlich um den Faktor 1,7 verglichen mit dem Beispiel 1: Harnstoffwäsche, Tropfenabscheider, Kristallisationsvorrichtung und Produktzyklon. Das Kreisgasgebläse (Fig. 1) kann hier vollständig entfallen.

### Bezugszeichenliste

- a): Wirbelschichtreaktor
- b): Heizelement
- c): Gasheizelement
- d): Gaskühler
- e): Gasfilter
- f): Kristallisationsvorrichtung
- g): Produktzyklon
- h): Kreisgasgebläse
- i): Harnstoffwäsche
- j): Kühleinheit
- k): Harnstoffvorratsbehälter
- I): Pumpe
- m): Tropfenabscheider
- n): Kompressor
- o): Feststoffkühler
- p): Quenchgasleitung

## Patentansprüche

1. Verfahren zur Herstellung von Melamin durch die Zersetzung von Harnstoff, **dadurch gekennzeichnet, dass** heißes, melaminhaltiges Reaktionsgas aus dem Reaktor zur Desublimation des Melamins in einem Kristallisationsgefäß durch gleichzeitiges Einleiten von kristallinem Melamin abkühlt (gequencht) wird, wobei das zum Quenchen eingesetzte, kristalline Melamin eine Temperatur von weniger als 100°C, vorzugsweise von etwa 40°C, aufweist.

2. Verfahren nach Anspruch 1, **dadurch gekennzeichnet, dass** man die melaminhaltigen Reaktionsgase in der Kristallisationsvorrichtung durch das Einleiten von kristallinem Melamin von etwa 350°C auf etwa 190°C abkühlt.

3. Verfahren nach den Ansprüchen 1 und 2, **dadurch gekennzeichnet, dass** die Temperatur des aus dem Kristallisationsgefäß entnommenen Melamins durch die Menge des zum Quenchen eingesetzten kristallinen Melamins reguliert wird, wobei ein vorgegebener Sollwert erreicht wird, der vorzugsweise nicht unter 190°C liegt.

4. Verfahren nach Anspruch 3, **dadurch gekennzeichnet, dass** der überwiegende Teil des aus dem Kristallisationsgefäß entnommenen Melamins im Kreislauf erneut in das Kristallisationsgefäß eingeführt und zum Quenchen der heißen, melaminhaltigen Reaktionsgase eingesetzt wird.

## Claims

1. A method of producing melamine through the decomposition of urea, **characterised in that** hot, melamine-containing reaction gas from the melamine-desublimation reactor is cooled (quenched)in a crystallisation vessel through the simultaneous introduction of crystalline melamine, wherein the crystalline melamine used for quenching exhibits a temperature of less than 100 °C, preferably of roughly 40 °C.

2. The method according to claim 1, **characterised in that** the melamine-containing reaction gases are cooled in the crystallisation device by introducing crystalline melamine from roughly 350 °C to roughly 190 °C.

3. The method according to claims 1 and 2, **characterised in that** the melamine removed from the crystallisation vessel is regulated by the amount of crystalline melamine used for quenching, wherein a predefined target value is achieved, which is preferably no lower than 190 °C.

4. The method according to claim 3, **characterised in that** the main part of the melamine removed from the crystallisation vessel is reintroduced into the crystallisation vessel during the cycle and used to quench the hot, melamine-containing reaction gases.

## Revendications

1. Procédé de préparation de mélamine par décomposition d'urée, **caractérisé en ce que** l'on refroidit un gaz de réaction chaud qui contient de la mélamine et provient du réacteur en l'introduisant, simultanément avec de la mélamine cristalline, dans un récipient de cristallisation afin d'y provoquer la condensation solide de la mélamine, la température de la mélamine cristalline mise en oeuvre pour le refroidissement étant inférieure à 100 °C, de préférence d'environ 40 °C.

2. Procédé selon la revendication 1, **caractérisé en ce que**, au sein du dispositif de cristallisation, on refroidit les gaz de réaction qui contiennent de la mélamine d'environ 350 °C à environ 190 °C, par l'introduction de mélamine cristalline.

3. Procédé selon les revendications 1 et 2, **caractérisé en ce que** la température de la mélamine que l'on récupère dans ledit récipient de cristallisation est régulée par la quantité de mélamine cristalline mise en oeuvre à des fins de refroidissement, de façon à ce que l'on atteigne une valeur consigne préalablement définie qui, de préférence, n'est pas inférieure à 190 °C.

4. Procédé selon la revendication 3, **caractérisé en ce que** la majeure partie de la mélamine récupérée dans ledit récipient de cristallisation est recyclée dans le récipient de cristallisation et mise en oeuvre pour le refroidissement desdits gaz de réaction chauds qui contiennent de la mélamine.
